(19) Europäisches Patentamt
European Patent Office
Office européen des brevets

(11) Veröffentlichungsnummer: **0 132 771**
**A2**

(12) # EUROPÄISCHE PATENTANMELDUNG

(21) Anmeldenummer: 84108443.7

(22) Anmeldetag: 18.07.84

(51) Int. Cl.$^4$: **C 07 D 249/08**
C 07 D 233/60, C 07 D 233/61
C 07 D 401/06, A 01 N 43/50
A 01 N 43/653

(30) Priorität: 22.07.83 DE 3326456
25.11.83 DE 3342671

(43) Veröffentlichungstag der Anmeldung:
13.02.85 Patentblatt 85/7

(84) Benannte Vertragsstaaten:
AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Schaller, Rainer, Dr.
Thyssenstrasse 31
D-8906 Gersthofen(DE)

(72) Erfinder: Ehrhardt, Heinz, Dr.
Bergstrasse 21
D-8901 Rehling(DE)

(72) Erfinder: Sachse, Burkhard, Dr.
An der Ziegelei 30
D-6233 Kelkheim (Taunus)(DE)

(72) Erfinder: Bürstell, Helmut, Dr.
Am Hohlacker 65
D-6000 Frankfurt am Main 50(DE)

(72) Erfinder: Bieringer, Hermann, Dr.
Eichenweg 26
D-6239 Eppstein/Taunus(DE)

(72) Erfinder: Bauer, Klaus, Dr.
Kolpingstrasse 7
D-6054 Rodgau(DE)

(54) **Neue 1,2 Diaryl-3-azolyl-propan-Derivate, ihre Herstellung und Verwendung als Pflanzenbehandlungsmittel.**

(57) Die neuen verbindungen der Formel I,

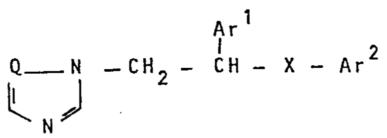
I

worin $Ar^1$ und $Ar^2$ Naphthyl, (subst.) Phenyl, einen (subst.) Thiophen-, (subst.) Furan- oder (subst.) Pyridin-Rest, Q N oder CH und X eine Gruppe

$>C{=}O$, $>C{=}S$, $>C{=}N{-}OR^3$, $>C(OR^3)R^7$, $>C(OR^8)_2$ oder

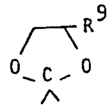

bedeuten, sowie ihre Salze, Metallsalzkomplexe und Quaternisierungsprodukte besitzen vorteilhafte fungizide, herbizide und wachstumsregulierende Eigenschaften.

EP 0 132 771 A2

Croydon Printing Company Ltd.

Neue 1,2-Diaryl-3-azolyl-propan-Derivate, ihre Herstellung und Verwendung als Pflanzenbehandlungsmittel

Gegenstand der Anmeldung sind neue 1,2-Diaryl-3-azolyl-propan-Derivate der Formel (I)

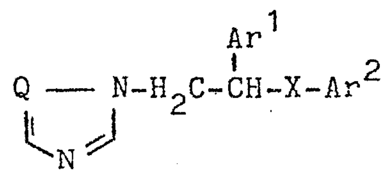 (I)

worin

$Ar^1$ und $Ar^2$ gleich oder verschieden sind und die Bedeutung von 1- oder 2-Naphthyl oder einer Gruppe

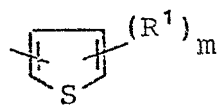 , 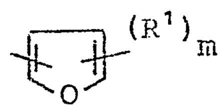 ,

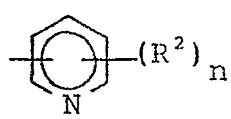 oder 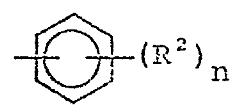

haben, mit

$R^1$ = Wasserstoff, $C_1$-$C_6$-Alkyl oder Halogen,

$R^2$ = Wasserstoff, Halogen, Halogen($C_1$-$C_4$)-alkyl, bevorzugt Trifluormethyl und Trichlormethyl, Nitril, Nitro, Hydroxy, ($C_1$-$C_6$)-Alkyl, ($C_5$-$C_6$)-Cycloalkyl, ($C_1$-$C_6$)-Alkoxy, Halogen($C_1$-$C_4$)-alkoxy, Phenoxy, Phenyl- oder Phenyl($C_1$-$C_4$)-alkyl, wobei die drei letztgenannten Reste bis zu 3 Halogenatome tragen können,

m = 1, 2 oder 3 und
n = 1, 2, 3 oder 4, wobei im Falle m > 1 oder n > 1 die Substituenten verschieden sein können,

X einen Rest der Formeln

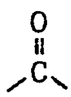 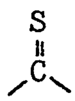 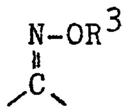 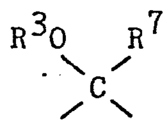 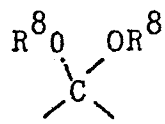

oder

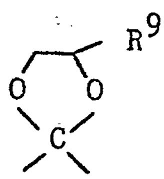

wobei in diesen Gruppierungen
$R^3$ Wasserstoff, $(C_1-C_6)$-Alkyl, $(C_5-C_6)$-Cycloalkyl, $(C_2-C_6)$-Alkenyl, $(C_3-C_6)$-Alkinyl, Phenyl-$(C_1-C_4)$-alkyl, Phenoxy-$(C_1-C_6)$-alkyl, wobei die beiden letztgenannten Gruppen ihrerseits bis zu dreifach halogensubstituiert sein können, oder einen Rest der Formeln

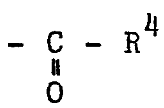 oder 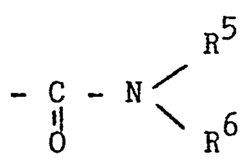 ,

wobei
$R^4$ $(C_1-C_6)$-Alkyl, $(C_5-C_6)$-Cycloalkyl, $(C_2-C_6)$-Alkenyl, Phenyl, Naphthyl oder Phenyl$(C_1-C_4)$-alkyl bedeutet, wobei die drei letztgenannten Reste bis zu dreifach durch Halogen, $(C_1-C_4)$-Alkyl oder $(C_1-C_4)$-Alkoxy oder durch eine

Trifluor- oder Trichlormethylgruppe substituiert sein können,

$R^5$,$R^6$ unabhängig voneinander Wasserstoff, ($C_1$-$C_6$)-Alkyl oder Phenyl, das durch Halogen substituiert sein kann, bedeuten, wobei im Fall $R^5$ oder $R^6$ Wasserstoff oder Phenyl bedeutet, $R^5$ und $R^6$ verschieden sein müssen,

$R^7$ Wasserstoff, ($C_1$-$C_6$)-Alkyl oder Phenyl, das ein bis dreifach halogeniert sein kann, bevorzugt Wasserstoff, wenn $R^3$ nicht Wasserstoff bedeutet,

$R^8$ bei gleicher Bedeutung ($C_1$-$C_6$)-Alkyl und

$R^9$ Wasserstoff, ($C_1$-$C_6$)-Alkyl, halogeniertes ($C_1$-$C_6$)-Alkyl, Phenyl, Naphthyl, Phenyl-($C_1$-$C_4$)-alkyl oder Phenoxy-($C_1$-$C_6$)-alkyl, wobei diese drei letztgenannten Reste ein- bis dreifach halogeniert sein können, und

Q N oder CH, bedeutet, wobei jedoch CH ausgenommen ist, wenn $Ar^1$ Phenyl und zusätzlich X einen Rest der Formel $>C=O$ oder $>CH-OR^{10}$ bedeutet, worin $R^{10}$ Wasserstoff oder einen Rest der Formel $-\underset{\overset{\|}{O}}{C}-R^{11}$ bedeutet, worin $R^{11}$ die Bedeutung von Phenyl, ($C_1$-$C_6$)-Alkyl oder von ($C_1$-$C_6$)-Dialkylamino hat,

sowie ihre Salze, Metallsalzkomplexe und Quaternisierungsprodukte.

Ein weiterer Gegenstand der Erfindung sind Verfahren zur Herstellung der neuen 1,2-Diaryl-3-azolylpropane der Formel I, ihrer Salze, Metallsalzkomplexe und Quaternisierungs-

produkte, sowie die Verwendung der neuen Substanzen als Pflanzenbehandlungsmittel.

Die Verfahren zur Herstellung der Verbindungen der Formel I sind dadurch gekennzeichnet, daß man

a) Verbindungen der Formel II,

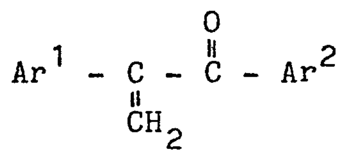

(II)

mit Imidazol oder 1,2,4-Triazol unter Zusatz einer Base umsetzt, oder

b) Verbindungen der Formel III,

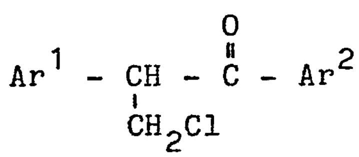

(III)

mit Imidazol oder 1,2,4-Triazol in Gegenwart eines Säurebindemittels umsetzt,

und die so erhaltenen Verbindungen der Formel I mit X=$\rangle$C=O gegebenenfalls durch Derivatisierung der Carbonylgruppe in andere Verbindungen der Formel I umwandelt und die erhaltenen Verbindungen der Formel I in ihre Salze, Metallsalzkomplexe und Quaternisierungsprodukte überführt.

Die Derivatisierung der Verbindungen der Formel I mit X=$\rangle$C=O erfolgt nach bekannten und üblichen Methoden. So werden die Verbindungen der Formel (I) mit X=$\rangle$C=S, in der Weise erhalten, daß man z.B. gemäß J.W. Scheeren et al. Synthesis 1973, 149, Verbindungen der Formel I mit X=$\rangle$C=O mit einem Schwefelungsreagenz, wie z.B. $P_4S_{10}$ (bevorzugt doppelt molare Menge) in Gegenwart einer Base, beispielsweise einem tertiären organischen Amin wie Triethylamin oder Pyridin oder einem Alkalihydrogencarbonat wie Natriumhydrogencarbonat in einem inerten Lösungsmittel, wie z.B. Toluol oder Xylol, bei Temperaturen zwischen 50 und 120°C, vorzugsweise bei 90 bis 105°C, umsetzt. Bei Verwendung von Pyridin als Base kann auch dieses selbst als Lösungsmittel dienen.

Erfindungsgemäße Oxime (Formel (I) mit X=$\rangle$C=N-OH) erhält man aus den entsprechenden Ketonen, beispielsweise gemäß der Vorschrift von A. Saednya, Synthesis 1982, 190-191, durch Umsetzung mit einem Überschuß an Hydroxylaminhydrochlorid, wobei dieser Überschuß etwa die doppeltmolare Menge betragen kann, in Gegenwart mindestens eines Moläquivalents eines Säurebindemittels, beispielsweise eines tertiären organischen Amins wie Triethylamin oder Pyridin, in einem inerten Lösungsmittel, beispielsweise Toluol und Xylol, oder auch ohne Lösungsmittel im Temperaturbereich von 50 bis 140°C, bevorzugt 90 bis 115°C. Überraschenderweise tritt hierbei keine Eliminations-Additionsreaktion zu N,N-Bis-(3-oxo-propyl)hydroxylamin (vgl. J.F. Hansen et al., J.Org.Chem. 44, 661 (1979)) ein, wie sie üblicherweise bei Mannich-Basen beobachtet wird.

Die Verbindungen der Formel (I) mit X=$\rangle$CH-OH) können durch Reduktion der Ketone nach prinzipiell bekannten Methoden, beispielsweise unter Verwendung von komplexen Metallhydriden in geeigneten Lösungsmitteln, wie z.B. Natriumborhydrid in Methanol, vorzugsweise bei Temperaturen zwischen 20 und 60°C, erhalten werden.

Verbindungen der Formel I mit X=$\rangle$COH($R^7$) erhält man durch Umsetzen der Ketone (Verbindungen der Formel I mit X=$\rangle$C=O mit dem entsprechenden Grignard-Reagenz der Formel $R^7MgX'$ (X'= Cl, Br, J, bevorzugt J) in einem etherischen Lösungsmittel wie Diethyläther, Tetrahydrofuran bei Temperaturen zwischen 10 und 40°C und nach folgender Hydrolyse.

Ketale der Formel (I) mit X= $C(OR^8)_2$ oder 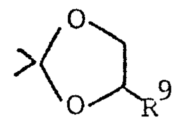

werden aus den Ketonen der Formel I (X=>CO) durch Umsetzung mit einem großen Überschuß des entsprechenden mono- oder bifunktionellen Alkohols in Gegenwart von mindestens 1,2 Moläquivalent Säure, wie z.B. p-Toluolsulfonsäure, und gleichzeitiger, kontinuierlicher Wasserauskreisung - z.B. unter Verwendung eines Wasserabscheiders oder durch Zusatz eines Molekularsiebes 4 Å, hergestellt.

Zur weiteren Derivatisierung können die Verbindungen der Formel (I) mit X=>C=N-OH oder >$COH(R^7)$ in bekannter Weise alkyliert, acyliert oder carbamoyliert werden.

Die Herstellung der Verbindungen der Formel (I) mit X=>C=O gemäß Verfahrensvariante a) erfolgt nach an sich bekannten Methoden. Die Umsetzungen werden vorteilhaft in Anwesenheit eines inerten Lösungsmittels, beispielsweise eines Aromaten wie Toluol oder Xylol, unter Zusatz katalytischer Mengen von nicht nucleophilen Basen, wie z.B. DBU (1,8-Diazabicyclo[5.4.0]undec-7-en) bei Temperaturen von 0°C bis 120°C durchgeführt, vorzugsweise arbeitet man bei 30 - 80°C.

Die Verbindungen der Formel II erhält man nach bekannten Verfahren (Chem. Ber. 89, 27 (1956) aus den nach ebenfalls bekannten Methoden darstellbaren Desoxybenzoinen (Houben-Weyl VII/2a, S. 135 ff. oder J.Amer.Chem.Soc, 72, 1990 (1950)) durch Umsetzung mit 1-2 Äquivalenten Formaldehyd in dipolaren Lösungsmitteln, wie beispielsweise Ethylenglykol, Dimethylformamid oder Methanol bei erhöhten Temperaturen in Gegenwart katalytischer Mengen eine Base wie Piperidin.

Die Umsetzungen zur Herstellung der Verbindungen der Formel I mit X=>CO gemäß Variante b) werden nach bekannten Methoden in Anwesenheit eines inerten Lösungsmittels und einer äquimolaren Menge eines Säurebindemittels bei er-

höhten Temperaturen - bevorzugt zwischen 40°C - 100°C durchgeführt. Als Lösungsmittel eignen sich beispielsweise Ketone wie Aceton oder Methylethylketon, Kohlenwasserstoffe, wie Toluol oder Xylol, polar aprotische Lösungsmittel, wie Dimethylformamid, N-Methylpyrrolidon oder Dimethylsulfoxid, oder auch Acetonitril. Als Säurebindemittel kommen organische oder anorganische Basen, wie Alkali- oder Erdalkalicarbonate oder deren Hydroxyde, sowie organische Amine wie Trialkylamine oder Pyridin, zur Anwendung.

Die an sich bekannten Verbindungen der Formel III (Bull. Soc.Chim.Fr. 1963 (6) 1176-81) erhält man nach üblichen Methoden aus ß-Chlor-1,2-diarylacroleinen (Chem.Ztg. 98, 606 (1974)) oder auch über eine Enoletherspaltung durch Behandeln der 3-Methoxy-prop-2-en-1-ole der Formel (IV)

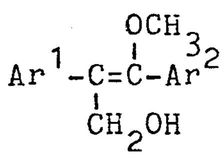

(IV)

mit 1,2 - 2,5 Moläquivalenten Thionylchlorid in inerten Lösungsmitteln beispielsweise chlorierten Kohlenwasserstoffen wie Tetrachlorkohlenstoff bei Temperaturen zwischen 60 - 80°C.

Die nach den beschriebenen Verfahren hergestellten Verbindungen der Formel I sind als basische Verbindungen zur Bildung von Salzen, Komplexsalzen und Quaternisierungsprodukten befähigt. Genannt seien die Salze von organischen und anorganischen Säuren, wie Benzoate, Fumarate, Oxalate,

Phenolate, Sulfonate, Nitrate, Chloride, Bromide und Sulfate, Komplexe mit Metallen der Gruppen I b, II b, IV b oder VIII des Periodensystems, etwa Kupfer, Zink und Zinn und Quaternisierungsprodukte mit Alkyl - insbesondere $(C_1-C_6)$-Alkyl - und gegebenenfalls im Phenylrest substituierten, insbesondere halogenierten Phenacylhalogeniden. Die Herstellung derartiger Derivate erfolgt nach den allgemein üblichen Methoden.

Verbindungen der allgemeinen Formel (I), ihre Salze, Komplexsalze und Quaternisierungsprodukte zeichnen sich durch eine ausgezeichnete fungizide Wirkung aus. Pilzliche Krankheitserreger, die in das pflanzliche Gewebe eindringen oder bereits eingedrungen sind, lassen sich mit ihrer Hilfe erfolgreich kurativ bekämpfen. Dies ist besonders vorteilhaft bei solchen Pilzerkrankungen, die nach erfolgter Infektion mit den üblichen, prophylaktisch wirkenden Fungiziden nicht mehr bekämpft werden können. Das Wirkungsspektrum der beanspruchten Verbindungen erfaßt z. B. neben verschiedenen Rostpilzen wie z. B. Puccinia recondita auch Piricularia oryzae, Venturia inaequalis, Cercospora beticola und Plasmopara viticola. Besonders hervozuheben ist die gute Wirkung der beanspruchten Verbindungen bei Echten Mehltaupilzen im Obst-, Gemüse-, Getreide- und Zierpflanzenbau.

Die Verbindungen der Formel (I) eignen sich auch für den Einsatz im technischen Bereich, beispielsweise in Holzschutzmitteln, auf dem Anstrichfarbensektor, als Konservierungsmittel, z. B. in Kühlschmiermitteln für die Metallbearbeitung.

Die erfindungsgemäßen Verbindungen eignen sich darüberhinaus zur selektiven Bekämpfung zweikeimblättriger und grasartiger annueller und perennierender Unkräuter in

landwirtschaftlich bedeutenden Kulturen wie z. B. Weizen, Gerste, Roggen, Mais, Zuckerrübe und Soja im Vorsaat-, Vorauflauf- und Nachauflaufverfahren.

Besonders gute herbizide Wirksamkeit wird erreicht, wenn die erfindungsgemäßen Verbindungen im Wasserreisanbau eingesetzt werden. Sie zeigen unter diesen Bedingungen eine breite Wirkung gegen eine Reihe von Reisunkräutern wie z. B. gegen Arten aus der Gruppe der Cyperaceen z. B. Eleoacharis, Cyperus, Scirpus oder gegen Arten wie Sagittaria, Pistia und gegen dikotyle Unkräuter. Die Kulturpflanze Reis toleriert die erfindungsgemäßen Verbindungen völlig, so daß diese zur selektiven Bekämpfung von Unkräutern in Reis im Vorauflauf- und im Nachauflaufverfahren eingesetzt werden können.

Kommen die Mittel in subtoxischen Dosen zur Anwendung, so weisen sie hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Baumwolle, Rasen sowie ihre Fähigkeiten, den Gehalt an erwünschten Inhaltsstoffen wie Kohlehydraten und Protein bei Nutzpflanzen zu erhöhen. Schließlich zeigen die Verbindungen eine sehr gute Verbesserung der Fruchtabszission, insbesondere bei Zitrusfrüchten,

...

oder Reduktion der Haltekraft.

Bevorzugt sind für die herbizide und wachstumsregulierende Anwendung solche Verbindungen der Formel I, worin Q=N; $Ar^1$ und $Ar^2$ Reste der Formel

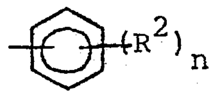 ,

X Reste der Formeln

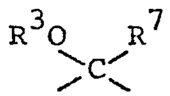 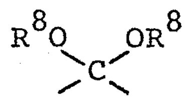 oder 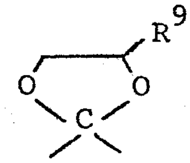 ,

$R^3$ Wasserstoff, $(C_1-C_4)$Alkyl, Phenoxy$(C_1-C_6)$alkyl, das bis zu dreifach halogeniert sein kann, oder -CO-Alkyl$(C_1-C_4)$, $R^7$ Wasserstoff oder $(C_1-C_4)$Alkyl, $R^8$ $(C_1-C_4)$Alkyl und $R^9$ Wasserstoff oder $(C_1-C_4)$Alkyl bedeuten.

Gegenstand der Erfindung sind auch fungizide, herbizide und pflanzenwachstumsregulierende Mittel, welche die Verbindungen der Formel I oder ihre Salze, Metallsalzkomplexe und Quarternisierungsprodukte enthalten.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6-disulfonsaures Natrium, dibutylnaphthalinsulfonsaures Natrium oder auch oleoylmethyltaurinsaures Natrium enthalten. Ihre Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitan-fettsäureester oder Polyoxethylensorbitester.

Stäubemittel kann man durch Vermahlen des Wirkstoffes mit fein verteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen

enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt, und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel-, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich; dabei können gegebenenfalls auch synergistische Wirkungssteigerungen erzielt werden.

Die Anwendungskonzentrationen der erfindungsgemäßen Verbindungen in der fertigen Spritzbrühe betragen bei der Verwendung als Fungizide im allgemeinen zwischen 1 mg und 500 mg Wirkstoff pro Liter Spritzbrühe (entsprechend 1 - 500 g Wirkstoff pro ha). Die Pflanzen werden hierbei tropfnaß gespritzt.

Bei der Verwendung als Herbizide oder wachstumsregulierende Mittel liegen die Aufwandmengen in der Größenordnung von 0,05 bis 5 kg Wirkstoff/ha. Üblicherweise erfolgt die Ausbringung der erfindungsgemäßen Mittel mit einem Wasservolumen von 20 - 1000 l/ha.

Die folgenden Beispiele dienen zur Erläuterung der Erfindung.

A. Formulierungsbeispiele

Beispiel A

Ein Stäubemittel wird erhalten, indem man 10 Gewichtsteile Wirkstoff mit 90 Gewichtsteilen Talkum als Inertstoff mischt und in einer Schlagmühle zerkleinert.

Beispiel B

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Beispiel C

Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Beispiel D

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

B. Chemische Beispiele

Herstellung der Ausgangsmaterialien

Beispiel I

3-(4-Chlorphenyl)-2-(2,4-dichlorphenyl)-3-methoxy-propenal

11,5 g (0,5 mol) Natrium wird in 375 ml abs.[1] Methanol eingetragen. In die heiße Lösung gibt man 173 g 3-Chlor-3-(4-chlorphenyl)-2-(2,4-dichlorphenyl)-propenal (Synthese nach Z. Arnold u. J. Zemlička, Proc. Chem. Soc. 1958, 227 und Coll. Czech. Chem. Commun. 24 (1959) 2385) in kleinen Protionen unter Feuchtigkeitsausschluß und hält dann die anfangs gelb-braune Suspension unter kräftigem Rühren bei Rückflußtemperatur. Allmählich entsteht eine klare Lösung, aus der beim Abkühlen ein kristalliner Niederschlag ausfällt. Der Niederschlag wird abfiltriert, gründlich mit Wasser gewaschen und getrocknet. Man erhält 166 g (96 % d. Theorie) an 3-(4-Chlorphenyl)-2-(2,4-dichlorphenyl)-3-methoxy-propenal mit einem Fp von 137 - 140 °C.

[1] abs. = wasserfrei

Beispiel II

3-(4-Chlorphenyl)-2-(2,4-dichlorphenyl)-3-methoxy-prop-2-en-1-ol

266 g (0,8 mol) 3-(4-Chlorphenyl)-2-(2,4-dichlorphenyl)-3-methoxy-propenal wird in abs. Methanol suspendiert und portionsweise mit 15 g (0,4 mol) Natriumborhydrid versetzt. Unter Gasabspaltung und leicht exothermer Reaktion entsteht eine klare Lösung. Man heizt zum Rückfluß bis die Gasabspaltung beendet ist, engt zur Trockene ein, nimmt mit Wasser/$CH_2Cl_2$ auf, trennt die organische Phase ab und engt sie nach dem Trocknen über $Na_2SO_4$ wieder ein. Man erhält so 253 g (96 % d. Theorie) an 3-(4-Chlorphenyl)-2-(2,4-dichlorphenyl)-3-methoxy-prop-2-en-1-ol als öliges Produkt von ca. 95%iger Reinheit.

Beispiel III

3-Chlor-1-(4-chlorphenyl)-2-(2,4-dichlorphenyl)-prop-1-on

100 g (0,3 mol) 3-(4-Chlorphenyl)-2-(2,4-dichlorphenyl)-3-methoxy-prop-2-en-1-ol werden in 250 ml Tetrachlorkohlenstoff gelöst und bei 0 - 5 °C mit 53,5 g (0,45 mol) Thionylchlorid versetzt. Man rührt bis zum Ende der Gasentwicklung bei Raumtemperatur nach und zieht im Wasserstrahlvakuum die bis 60 °C Badtemperatur flüchtigen Bestandteile ab. Man erhält 97,6g(98 % d. Theorie) an 3-Chlor-1-(4-chlorphenyl)-2-(2,4-dichlorphenyl)-propan-1-on.

Beispiel IV

2-(2,4-Dichlorphenyl)-3-(4-fluorphenyl)-prop-1-en-3-on

28,3 g (0,1 mol) (2,4-Dichlorbenzyl)-(4-fluorphenyl)-keton in 80 ml Dimethylformamid werden mit 30 ml (0,3 mol) 30%iger Formaldehyd-Lösung auf 120 °C erhitzt. Innerhalb 5 min läßt man zur heißen Lösung 0,5 ml Piperidin in 10 ml Methanol zutropfen. Nach ca. 6 h entsteht aus der Aufschlämmung eine klare Lösung. Man läßt abkühlen und versetzt mit Wasser - eine ölige Schicht trennt sich ab. Sie wird mit $CH_2Cl_2$ verdünnt, mit verd. Schwefelsäure, Bicarbonat-Lösung und Wasser gewaschen, über Natriumsulfat getrocknet und eingedampft. Im Hochvakuum werden Spuren von DMF[2)] entfernt. Man erhält 28,9 g (98 % d.Th.) an 2-(2,4-Dichlorphenyl)-3-4-fluorphenyl)-prop-1-en-3-on als 95%-iges öliges Produkt

[2)] Dimethylformamid

Herstellung der Verbindungen der Formel I

Beispiel 1 (Verfahren a))

1,2-Diphenyl-3-(1,2,4-triazol-1-yl)-propan-1-on

14,6 g (0,07 mol) α-Methylendesoxybenzoin und 6,22 g (0,09 mol) Triazol werden in abs. Toluol nach Zusatz katalytischer Mengen DBU unter starkem Rühren ca. 5 Tage bei 50 - 60 °C gerührt. Das Edukt ist dann lt. DC-Probe vollständig verbraucht. Zur Aufarbeitung schüttelt man mit Ammonchlorid/Wasser aus, trocknet die organische Phase über $Na_2SO_4$ und rotiert ein. Man erhält nach Anreiben mit Diisopropylether 19 g (98 % d. Th.) 1,2-Diphenyl-3-(1,2,4-triazol-1-yl)-propan-1-on vom Fp 114 - 115 °C.

Beispiel 2 (Verfahren b))

2-(4-Chlorphenyl)-1-phenyl-3-(1,2,4-triazol-1-yl)-propan-1-on

22,8 g (0,33 mol) Triazol und 33,4 g (0,33 mol) Triethylamin werden in abs. Toluol aufgeschlämmt und bei Raumtemperatur mit einer Toluollösung von 83,7 g (0,3 mol) 3-Chlor-2-(4-chlorphenyl)-1-phenyl-propan-1-on versetzt. Nach Erwärmen auf 40 - 50 °C geht das Triazol deutlich vermehrt in Lösung, während gleichzeitig Ammoniumsalze ausfallen. Man rührt ca. 5 h bis zum Verbrauch des Edukts. Man filtriert ab, wäscht den Rückstand gründlich mit Wasser und erhält so einen Teil des Produktes. Weiteres Produkt wird nach Behandeln der Mutterlauge auf übliche Weise isoliert. Man erhält so 91,6 g (98 % d. Th.) an 2-(4-Chlorphenyl)-1-phenyl-3-(1,2,4-triazol-1-yl)-propan-1-on vom Fp 110 - 115 °C.

Beispiel 3

1,2-Diphenyl-3-(1,2,4-triazol-1-yl)-thio-propanon-(1)

15 g (0,05 mol) 1,2-Diphenyl-3-(1,2,4-triazol-1-yl)-propanon-(1) werden zusammen mit 11,1 g (0,025 mol) Phosphorpentasulfid in 100 ml wasserfreiem Pyridin

aufgeschlämmt und zum Rückfluß erhitzt. Nach ca. 3 h entsteht eine klare Lösung, die nach dem Abkühlen mit ca. 200 ml Ethanol versetzt und noch weitere 5 h zum Rückfluß erhitzt wird. Die Aufarbeitung erfolgt durch Eindampfen zur Trockene und Extrahieren des Rückstandes mit Wasser/Methylenchlorid. Nach Trocknen und Eindampfen der organischen Phase erhält man 12,4 g (85 % d. Th.) 1,2-Diphenyl-3-(1,2,4-triazol-1-yl)-thiopropanon (1) vom Fp 99 - 101 °C.

Beispiel 4

1,2-Diphenyl-3-(1,2,4-triazol-1-yl)-propan-1-on-oxim

20 g 1,2-Diphenyl-3-(1,2,4-triazol-1-yl)-propanon-(1) wird in 11,5 g Pyridin gelöst. Man gibt vorsichtig 5,1 g Hydroxyl-amin-hydrochlorid zu und rührt ca. 10 min nach. Die entstandene Aufschlämmung wird mit 265 ml Toluol versetzt und am Wasserabscheider zum Sieden erhitzt. Zur Aufarbeitung filtriert man vom entstandenen Niederschlag ab, wäscht diesen mit Wasser und kristallisiert den unlöslichen Anteil aus Butanol um; man erhält 17,1 g (82 % d. Th.) 1,2-Diphenyl-3-(1,2,4-triazol-1-yl)-propan-1-on-oxim vom Fp 208-211 °C.

Beispiel 5

2-(4-Chlorphenyl)-2-[(2-imidazol-1-yl-1-phenyl)-ethyl]-1,3-dioxolan

31,1 g 1-(4-Chlorphenyl)-3-imidazol-1-yl-2-phenyl-propan-(1) werden in 200 ml wasserfreiem Toluol aufgeschlämmt. Man leitet trockenes HCl-Gas bis zur Sättigung ein, setzt 12,4 g Ethylenglykol und 34,4 g p-Toluol-sulfonsäure-monohydrat zu und kocht 16 h am Wasserabscheider. Hierauf werden noch einmal 15 ml Ethylenglykol zugegeben; sodann fügt man ca. 10 g Molekular-

sieb 4 Å zu und erhitzt 19 h am Rückfluß. Zur Aufarbeitung filtriert man vom Niederschlag ab und wäscht diesen mit Essigester. Der Niederschlag wird mit $K_2CO_3$-haltigem Wasser/Methylenchlorid behandelt und die organische Phase über $Na_2SO_4$ getrocknet und eingedampft. Man erhält 25,6 g (72 % d. Theorie) 2-(4-Chlorphenyl)-2-[(2-imidazol-1-yl-1-phenyl)-ethyl]-1,3-dioxolan vom Fp 144 - 146 °C.

Beispiel 6

1,2-Diphenyl-3-(1,2,4-triazol-1-yl)-propanol(1)

13,8 g 1,2-Diphenyl-3-(1,2,4-triazol-1-yl)-propanon-(1) werden in 50 ml Methanol aufgeschlämmt. Die portionsweise Zugabe von 0,9 g Natriumborhydrid führt zu einer exothermen Reaktion. Es entsteht eine klare Lösung aus der beim Abkühlen ein Niederschlag ausfällt. Zur Vervollständigung der Umsetzung hält man das Reaktionsgemisch noch 3 h bei ca. 40 °C und filtriert den farblosen Niederschlag ab. Man wäscht diesen gründlich mit Wasser und trocknet ihn bei 40 °C im Vakuum. Man erhält 11,7 g (85 % d. Theorie) 1,2-Diphenyl-3-(1,2,4-triazol-1-yl)-propanol(1) vom Fp 158 - 162 °C.

Beispiel 7

2,3-Diphenyl-4-(1,2,4-triazol-1-yl)-butanol-(2)

11,0 g 1,2-Diphenyl-3-(1,2,4-triazol-1-yl)-propanon(1) werden in eine aus 1,90 g Mg-Spänen und 11,4 g Methyljodid in 80 ml abs. Ether bereitete Grignard-Lösung portionsweise unter Feuchtigkeitsausschluß eingetragen. Die Temperatur wird bei 20 bis 30 °C gehalten. Zum Reaktionsgemisch werden einige Milliliter wasserfreies Tetrahydrofuran gegeben. Man erhitzt noch 2 h auf Rückflußtemperatur, versetzt das Reaktionsgemisch sodann mit 100 ml gesättigter, wäßriger Ammoniumchlorid-Lösung und trennt nach kräftigem Rühren die organische Phase

0132771

ab. Nach dem Trocknen über $Na_2SO_4$ destilliert man das Lösungsmittel im Vakuum ab und erhält 10,9 g (94 % d. Theorie) 2,3-Diphenyl-4-(1,2,4-triazol-1-yl)-butanol-(2) vom Fp 121 bis 129 °C.

Beispiele 8 bis 125

Nach den in den Beispielen 1 bis 7 angegebenen Vorschriften beziehungsweise durch literaturbekannte Derivatisierung der Verbindungen dieser Beispiele wurden die in nachstehender Tabelle 1 aufgelisteten Verbindungen der Formel (I) erhalten. Das Zeichen * in Spalte 6 der Tabelle gibt an, daß zur Herstellung der betreffenden Verbindung eine literaturbekannte Derivatisierung des betreffenden Beispiels durchgeführt wurde.

Tabelle 1

| Verb. gem. Bsp. | $Ar^1$ | $Ar^2$ | Q | X | Herst. gem. Bsp. | Fp °C |
|---|---|---|---|---|---|---|
| 8 | $3-Cl-C_6H_4$ | $4-F-C_6H_4$ | N | Et-C-OH | 7 | 62 |
| 9 | " | " | N | Et-C-OH 1) | 7* | 226 |
| 10 | $C_6H_5-$ | $4-Cl-C_6H_4$ | N | $CH_3-C-OH$ | 7 | Sirup |
| 11 | " | $C_6H_5$ | N | $>CH-OCOCH_2-O-$ 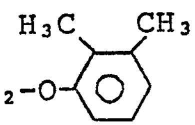 | 6* | 121-3 |
| 12 | " | " | N | $>CHOCH_2-C_6H_3-2,4-Cl_2$ | 6* | Sirup |
| 13 | " | $4-Cl-C_6H_4$ | N | $>CH-O(CH_2)_3O-C_6H_4-4-Cl$ | 6* | " |
| 14 | " | $4-CH_3-C_6H_4$ | CH | $CH_3-C-OH$ | 7 | " |
| 15 | " | $4-Cl-C_6H_4$ | N | $>CHOCH_2-O-C_6H_4-4-Cl$ | 6* | " |
| 16 | " | $4-CH_3-C_6H_4$ | N | $>CHOCOCH_3$ | 6* | Harz |
| 17 | $3-F-C_6H_4$ | $C_6H_5$ | N | $>C(CH_3)OH$ 1) | 7* | 152 |
| 18 | $2,4-Cl_2-C_6H_3$ | $4-Cl-C_6H_4$ | N | $>CHOH$ | 6 | 147-9 |
| 19 | $C_6H_5$ | $C_6H_5$ | CH | $>CHOCH_2C_6H_5$ | 6 * | Sirup |

Fortsetzung Tabelle 1

| Verb. gem. Bsp. | $Ar^1$ | $Ar^2$ | Q | X | Herst. gem. Bsp. | Fp °C |
|---|---|---|---|---|---|---|
| 20 | $C_6H_5$ | $4-CH_3-C_6H_4$ | N | >C=O | 1,2 | Sirup |
| 21 | " | $4-Cl-C_6H_4$ | N | >C=O | 1,2 | " |
| 22 | $2,4-Cl_2-C_6H_3$ | $C_6H_5$ | N | >C=O 1) | 1,2* | 226-30 |
| 23 | " | $4-CH_3-C_6H_4$ | N | >C=O 2) | 1,2* | 123-7 |
| 24 | " | $4-Cl-C_6H_4$ | N | >C=O | 1,2 | Sirup |
| 25 | " | " | N | >C=O 2) | 1,2* | 134-43 |
| 26 | $3-F-CH_{6\,4}$ | $C_6H_5$ | N | >C=O | 1,2 | 82-3 |
| 27 | $C_6H_5$ | $4-F-C_6H_4$ | N | >C=O | 1,2 | Sirup |
| 28 | $4-Cl-C_6H_4$ | $C_6H_5$ | N | $CH_3-C-OH$ | 7 | Sirup |
| 29 | " | " | N | " 1) | 7* | 152 |
| 30 | $C_6H_5$ | $4-Cl-C_6H_4$ | N | >CHOH | 6 | Sirup |
| 31 | " | $4-F-C_6H_4$ | N | $CH_3-C-OH$ | 7 | 136-9 |
| 32 | " | " | N | " 1) | 7* | 152 |
| 33 | " | $4-CH_3-C_6H_4$ | N | >CHOH | 6 | Sirup |
| 34 | " | $C_6H_5$ | N | $>CHOC(=O)NH-C_6H_4-4-Cl$ | 6* | 121-3 |
| 35 | $2-Cl-C_6H_4$ | $4-Cl-C_6H_4$ | N | $CH_3-C-OH$ 2) | 7* | 120-4 |

...

Fortsetzung Tabelle 1

| Verb. gem. Bsp. | $Ar^1$ | $Ar^2$ | Q | X | Herst. gem. Bsp. | Fp °C |
|---|---|---|---|---|---|---|
| 36 | $2-Cl-C_6H_4$ | $C_6H_5$ | N | 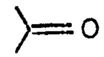 | 1,2 | Harz |
| 37 | " | $4-F-C_6H_4$ | N | 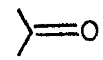 | 1,2 | " |
| 38 | $4-F-C_6H_4$ | $C_6H_5$ | N | " | 1,2 | 65-70 |
| 39 | $2,4-Cl_2-C_6H_3$ | $4-F-C_6H_4$ | N | " | 1,2 | Harz |
| 40 | $2-F-C_6H_4$ | $C_6H_5$ | N | " | 1,2 | Sirup |
| 41 | $2,4-Cl_2-C_6H_3$ | $4-F-C_6H_4$ | N | "4) | 1,2* | 161 |
| 42 | $4-Cl-C_6H_4$ | $C_6H_5$ | N | >CHOH | 6 | 148 |
| 43 | $2-Cl-C_6H_4$ | $C_6H_5$ | N | $-C-CH_3$<br>OH | 7 | 158-163 |
| 44 | " | $4-F-C_6H_4$ | N | >CHOH | 6 | Sirup |
| 45 | $C_6H_5$ | $4-CH_3-C_6H_4$ | N | $>CHO(CH_2)_2O-C_6H_3-2,4-Cl_2$ | 6* | " |
| 46 | $4-F-C_6H_4$ | $C_6H_5$ | N | 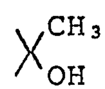 | 7 | " |
| 47 | $4-Cl-C_6H_4$ | " | N | $>CHOCH_2O-C_6H_3-2,4-Cl_2$ | 6* | 167 |
| 48 | $2,4-Cl_2-C_6H_3-$ | $C_6H_5$ | N | 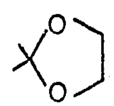 | 5 | 112-6 |

0132771

Fortsetzung Tabelle 1

| Verb. gem. Bsp. | $Ar^1$ | $Ar^2$ | Q | X | Herst. gem. Bsp. | Fp °C |
|---|---|---|---|---|---|---|
| 49 | $2-Cl-C_6H_4$ | $4-Cl-C_6H_4$ | N | $>CHOCH_2OC_6H_3-2,4-Cl_2$ | 6* | Sirup |
| 50 | α-Naphthyl- | $2,4-Me_2-C_6H_3$ | N | 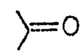 | 1,2 | " |
| 51 | $3-Cl-C_6H_4$ | $C_6H_5$ | N | " | 1,2 | " |
| 52 | $C_6H_5$ | " | N | "2) | 1,2* | 193-6 |
| 53 | $2,4-Cl_2-C_6H_3$ | 3-Pyridyl- | N | " | 1 | Sirup |
| 54 | $4-Cl-C_6H_4$ | " | N | " | 1 | 141-51 |
| 55 | $2,4-Cl_2-C_6H_3$ | $C_6H_5$ | N | 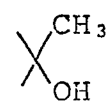 | 7 | Sirup |
| 56 | $2-Cl-C_6H_4$ | $4-F-C_6H_4$ | N | $>CHOCH_2O-C_6H_3-2,4-Cl_2$ | 6* | " |
| 57 | $2,4-Cl_2-C_6H_3$ | $4-F-C_6H_4$ | N | $>CHOC(=O)CH_3$ | 6* | " |
| 58 | $C_6H_5$ | $C_6H_5$ | N | 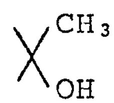 5) | 7* | Harz |
| 59 | $4-Cl-C_6H_4$ | " | N | " 3) | 6* | 222 |
| 60 | $2,4-Cl_2-C_6H_3$ | " | N | >CHOH 1) | 6* | 246 |
| 61 | $2-Cl-C_6H_4$ | $4-Me-C_6H_4$ | N | 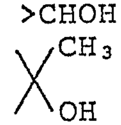 | 7 | 107-20 |

Fortsetzung Tabelle 1

| Verb. gem. Bsp. | $Ar^1$ | $Ar^2$ | Q | X | Herst. gem. Bsp. | Fp °C |
|---|---|---|---|---|---|---|
| 62 | $2-Cl-C_6H_4$ | $4-Me-C_6H_4$ | N | 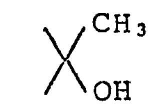 6) | 7* | 180-3 |
| 63 | " | $C_6H_5$ | CH | " | 7 | Sirup |
| 64 | $2,4-Cl_2-C_6H_3$ | $4-F-C_6H_4$ | N | " | 7* | 234-6 |
| 65 | $C_6H_5$ | $C_6H_5$ | CH | $>CHOCONH-C_6H_4-4-Cl$ | 6* | 142-8 |
| 66 | " | " | " | $>C=N-OH$ | 4 | 194-9 |
| 67 | $4-Cl-C_6H_4$ | " | N | $>CH-OCO-NH-C_6H_4-3-CF_3$ | 6* | 96 |
| 68 | $C_6H_5$ | " | CH | $>CH-OCH_2CON(CH_3)-C_6H_5$ | 6* | Sirup |
| 69 | " | " | " | >CHOCO-Imidazol-1-yl | 6* | 130-3 |
| 70 | " | " | N | 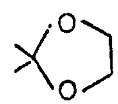 | 5 | 156-8 |
| 71 | " | " | CH | $>CHO(CH_2)_2O-C_6H_3-2,4-Cl_2$ | 6* | Harz |
| 72 | " | " | " | $>CHO(CH_2)_3O-C_6H_4-4-Cl$ | 6* | Sirup |
| 73 | $2-Cl-C_6H_4$ | $4-Cl-C_6H_4$ | N | >CHOH | 6* | " |
| 74 | " | $4-C_2H_5-C_6H_4$ | CH | >C(OH)- 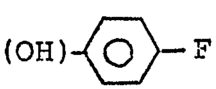 | 7 | 110 |

Fortsetzung Tabelle 1

| Verb. gem. Bsp. | $Ar^1$ | $Ar^2$ | Q | X | Herst. gem. Bsp. | Fp °C |
|---|---|---|---|---|---|---|
| 75 | $2,4-Cl_2-C_6H_3$ | $3,4-(CH_3)_2-C_6H_3$ | N | >C=N-OH | 4 | 170-3 |
| 76 | $C_6$[illegible] | $C_6H_5$ | N | >C (OH)-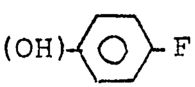 | 7 | 144-52 |
| 7[illegible] | " | " | CH | $>C=N-OCONH-C_6H_4-3-CF_3$ | 4* | Harz |
| [illegible] | $2-Cl-C_6H_4$ | " | N | >CH-OH | 6* | Sirup |
| 79 | $2,4-Cl_2-C_6H_3$ | $4-F-C_6H_4$ | CH | 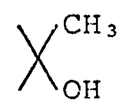 | 7 | 143 |
| 80 | $2-Cl-C_6H_4$ | $C_6H_5$ | " | >CH-OH | 6 | 153-5 |
| 81 | $2-F-C_6H_4$ | " | N | 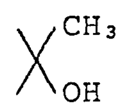 | 7 | Sirup |
| 82 | $2,4-Cl_2-C_6H_3$ | $4-F-C_6H_4$ | N | >CH-OH | 6 | " |
| 83 | $4-F-C_6H_4$ | $C_6H_5$ | N | >CHOH | 6 | " |
| 84 | $2,4-Cl_2-C_6H_3$ | $3,4(CH_3)_2-C_6H_3$ | CH | 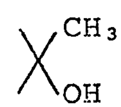 1) | 7* | 180-3 |
| 85 | $2-F-C_6H_4$ | $C_6H_5$ | N | >CHOH | 6 | 151-3 |

...

-25-

0132771

Fortsetzung Tabelle 1

| Verb. gem. Bsp. | $Ar^1$ | $Ar^2$ | Ω | X | Herst. gem. Bsp. | Fp °C |
|---|---|---|---|---|---|---|
| 86 | $2-Cl-C_6H_4$ | $4-C_2H_5-C_6H_4$ | N | 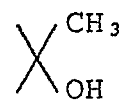 1) | 7* | 167-9 |
| 87 | $2,4-Cl_2-C_6H_3-$ | $3,4-(CH_3)_2-C_6H_3$ | N | >CHOH | 6 | Sirup |
| 88 | $2-Cl-C_6H_4$ | $4-CH_3-C_6H_4$ | N | 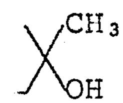 4) | 7* | 158-65 |
| 89 | " | $4-(i-C_3H_7)-C_6H_4$ | N | " | 7 | 134-44 |
| 90 | $4-Cl-C_6H_4$ | $4-CH_3-C_6H_4$ | N | 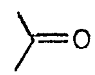 | 1,2 | 132-6 |
| 91 | " | $4-Cl-C_6H_4$ | N | " | 1,2 | Sirup |
| 92 | $2-Cl-C_6H_4$ | " | N | " | 1,2 | 127-30 |
| 93 | $4-Cl-C_6H_4$ | $C_6H_5$ | CH | " | 1,2 | 108-12 |
| 94 | " | $4-CH_3-C_6H_4$ | CH | " | 1,2 | Sirup |
| 95 | " | $4-Cl-C_6H_4$ | CH | " | 1,2 | " |
| 96 | $2,4-Cl_2-C_6H_3$ | $C_6H_5$ | CH | " | 1,2 | " |
| 97 | " | $4-CH_3-C_6H_4$ | CH | " 2) | 1,2* | 202-5 |
| 98 | " | $4-Cl-C_6H_4$ | CH | " 2) | 1,2* | 188-9 |
| 99 | $2-Cl-C_6H_4$ | $C_6H_5$ | CH | " | 1,2 | 88-91 |
| 100 | $2,4-Cl_2-C_6H_3$ | $4-F-C_6H_4$ | CH | " | 1,2 | Harz |

Fortsetzung Tabelle 1

| Verb. gem. Bsp. | $Ar^1$ | $Ar^2$ | Q | X | Herst. gem. Bsp. | Fp °C |
|---|---|---|---|---|---|---|
| 101 | $2-Cl-C_6H_4$ | $C_6H_5$ | N | 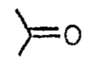 8) | 1,2* | 182-4 |
| 102 | $2,4-Cl_2C_6H_3$ | $3,4-(CH_3)_2-C_6H_3$ | N | " | 1,2 | Sirup |
| 103 | " | " | CH | " | 1,2 | " |
| 104 | $4-F-C_6H_4$ | $C_6H_5$ | N | " 4) | 1,2* | 135 |
| 105 | $2,4-Cl_2-C_6H_3$ | $4-C_6H_5-C_6H_4$ | N | " | 1,2 | Sirup |
| 106 | " | " | CH | " | 1,2 | " |
| 107 | $2-Cl-C_6H_4$ | $4-(i-C_3H_7)-C_6H_4$ | N | " | 1,2 | " |
| 108 | " | " | CH | " | 1,2 | " |
| 109 | " | $4-Et-C_6H_4$ | N | " | 1,2 | " |
| 110 | " | " | CH | " | 1,2 | " |
| 111 | " | $4-CH_3-C_6H_4$ | N | " | 1,2 | 108-11 |
| 112 | " | " | CH | " | 1,2 | Sirup |
| 113 | $3-Cl-C_6H_4$ | $4-F-C_6H_4$ | N | " | 1,2 | " |
| 114 | " | " | CH | " | 1,2 | " |
| 115 | $2-Cl-C_6H_4$ | $4-CH_3-C_6H_4$ | N | " 4) | 1,2* | 208-11 |
| 116 | $3-Cl-C_6H_4$ | $C_6H_5$ | N | " 3) | 1,2* | Sirup |
| 117 | $C_6H_5$ | $4-CH_3-C_6H_4$ | N | " 3) | 1,2* | " |

Fortsetzung Tabelle 1

| Verb. gem. Bsp. | $Ar^1$ | $Ar^2$ | Q | X | Herst. gem. Bsp. | Fp °C |
|---|---|---|---|---|---|---|
| 118 | $C_6H_5$ | $C_6H_5$ | CH | >CHO$(CH_2)_3$-O 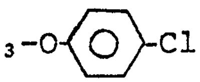 4) | 6* | 157-9 |
| 119 | " | " | N | >$CHOCH_2$ 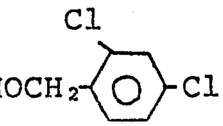 4) | 6* | 143 |
| 120 | " | " | N | 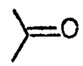 8) | 1,2* | 175 |
| 121 | 2-Cl-$C_6H_4$ | 4-$CH_3$-$C_6H_4$ | N | " 5) | 1,2* | 75-80 |
| 122 | 2-Cl-$C_6H_4$ | " | N | " 9) | 1,2* | 117-21 |
| 123 | " | " | N | 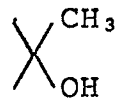 1) | 7* | 167-9 |
| 124 | $C_6H_5$ | 4-t-$C_4H_9$-$C_6H_4$ | N | 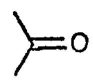 | 1,2 | Sirup |
| 125 | 2,6-$Cl_2$-$C_6H_3$ | $C_6H_5$ | N | " | 1,2 | 97-100 |

...

Erläuterungen zur Tabelle 1

1) Salz mit 1,5-Naphthalin-disulfonsäure
2) Salz mit p-Toluolsulfonsäure
3) Verbindung mit 4-Chlorphenacylbromid
4) Verbindung mit Methyljodid
5) Salz mit Tetrafluoroborsäure
6) Komplexsalze mit Kupfer-II-chlorid
7) Salz mit Salpetersäure
8) Salz mit Pikrinsäure
9) Salz mit Fumarsäure

Me = $CH_3$
Et = $C_2H_5$

B. Biologische Beispiele

1. Herbizide Wirkung im Vorauflaufverfahren

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt wurde. Dabei bedeutet:

0 = ohne Wirkung (Schaden)
1 = 0 - 20 % Wirkung
2 = 20 - 40 % Wirkung
3 = 40 - 60 % Wirkung
4 = 60 - 80 % Wirkung
5 = 80 - 100 % Wirkung

Samen bzw. Rhizomstücke mono- und dikotyler Unkräuter wurden in Lehmerde in Plastiktöpfen (Ø 9 cm) ausgelegt und mit Erde abgedeckt. Die als benetzbare Pulver bzw. als Emulsionskonzentrate formulierten erfindungsgemäßen Verbindungen wurden in Form wäßriger Suspensionen bzw. Emulsionen auf die Erdoberfläche appliziert. Die Wasseraufwandmenge pro Topf entsprach dabei umgerechnet 600 - 800 l/ha. Nach der Behandlung wurden die Versuchstöpfe im Gewächshaus aufgestellt und die Versuchspflanzen unter guten Wachstumsbedingungen (Temperatur: 23 ± 1 °C; rel. Luftfeuchte 60 - 80 %) kultiviert. Nach ca. 3 Wochen wurde die Pflanzenschädigung optisch bonitiert. Als Vergleich dienten dabei unbehandelte Kontrollen. Wie aus den Werten der Tabelle 2 hervorgeht, weisen die erfindungsgemäßen Verbindungen im Vorauflaufverfahren eine zum Teil ausgezeichnete herbizide Wirksamkeit gegen wirtschaftlich bedeutende mono- und dikotyle Schadpflanzen auf.

...

Tabelle 2

Herbizide Wirkung der erfindungsgemäßen Verbindungen im Vorauflauf (Dosis: 2,5 kg a. i./ha)

| Beispiel-Nr. | AMR | SIA | LOM | ECG | STM |
|---|---|---|---|---|---|
| 1 | 5 | - | 5 | 5 | 5 |
| 2 | 5 | 5 | 4 | 5 | - |
| 3 | 5 | 4 | - | 5 | - |
| 6 | 5 | 4 | 3 | 5 | 4 |
| 7 | 5 | 5 | 5 | 5 | 5 |
| 8 | 5 | 5 | 5 | 5 | 5 |
| 9 | 5 | 5 | 5 | 5 | 5 |
| 11 | 5 | 5 | - | 4 | 4 |
| 22 | 4 | 3 | 3 | 5 | 5 |
| 26 | 4 | 3 | - | 5 | 3 |
| 28 | 5 | 4 | 4 | 5 | 4 |
| 29 | 5 | 4 | 3 | 5 | 5 |
| 36 | 5 | 3 | - | 5 | 3 |
| 38 | 4 | 3 | - | 5 | - |
| 39 | 5 | - | - | 5 | 3 |
| 42 | 5 | 3 | 3 | 5 | 4 |
| 44 | 5 | 4 | 5 | 5 | 5 |
| 46 | 5 | - | 3 | 5 | 4 |
| 49 | 5 | 3 | - | 5 | 4 |
| 51 | 4 | 5 | - | 5 | 3 |
| 55 | 5 | 4 | 4 | 5 | 4 |
| 56 | 5 | 4 | 4 | 5 | 4 |
| 57 | 5 | 4 | 4 | 5 | 4 |
| 58 | 5 | 5 | 4 | 5 | 5 |
| 60 | 5 | 4 | 5 | 5 | 5 |
| 61 | 5 | 4 | 3 | 5 | 5 |
| 62 | 4 | 4 | - | 5 | 3 |
| 97 | 5 | - | - | - | - |
| 101 | 5 | - | - | 5 | 4 |

...

Fortsetzung Tabelle 2

| Beispiel-Nr. | AMR | SIA | LOM | ECG | STM |
|---|---|---|---|---|---|
| 113 | 5 | 3 | - | 5 | 4 |
| 123 | 4 | 3 | - | - | 5 |

STM = Stellaria media
AMR = Amaranthus retroflexus
SIA = Sinapis avensis
LOM = Lolium multiflorum
ECG = Echinochloa crus-galli
a.i.= Aktivsubstanz

2. Kulturpflanzenverträglichkeit bei Anwendung als Herbizid

Verschiedene Kulturpflanzen und Unkräuter wurden in sandigem Lehmboden in Töpfen von 9 cm Durchmesser ausgesät und im Vorauflaufverfahren mit den erfindungsgemäßen Verbindungen behandelt.
Die Töpfe wurden dann 4 Wochen lang im Gewächshaus unter günstigen Wachstumsbedingungen gehalten und Schädigungen der aufgelaufenen Kulturpflanzen und Unkräuter wurden im Vergleich zu Unbehandelt bonitiert (Tabelle 3).
Die in der Tabelle dargestellten Boniturergebnisse zeigen, daß die neuen Verbindungen von vielen Kulturpflanzen ohne wesentliche Schäden toleriert werden und verschiedene Unkräuter wie Setaria und Galium sehr gut bekämpfen.

...

Tabelle 3

Selektivität und herbizide Wirkung (%)

| Beispiel Nr. | | Weizen | Mais | Reis | Raps | Baumwolle | SAV | GAA |
|---|---|---|---|---|---|---|---|---|
| 1 | 2,4 | 0 | 10 | 0 | 0 | 15 | 100 | 100 |
| | 0,6 | 0 | 0 | 0 | 0 | 0 | 98 | 90 |
| 6 | 2,4 | 5 | 0 | 0 | 10 | 0 | 100 | 100 |
| | 0,6 | 0 | 0 | 0 | 0 | 0 | 95 | 92 |
| 58 | 2,4 | 30 | 45 | 0 | 5 | - | 100 | 100 |
| | 0,6 | 0 | 15 | 0 | 0 | - | 100 | 98 |
| 60 | 2,4 | 0 | - | 60 | 35 | 5 | 100 | 95 |
| | 0,6 | 0 | - | 10 | 10 | 0 | 100 | 90 |

SAV = Setaria viridis
GAA = Galium aparine

3. Herbizide Wirkung bei Anwendung in Reis

Knollen und Rhizome bzw. Jungpflanzen oder Samen verschiedener Reisunkräuter wie Cyperus-Arten, Eleocharis, Scirpus und Echinochloa werden in geschlossenen Töpfen von 13 cm Durchmesser ausgepflanzt und mit Wasser bis zur Höhe von 1 cm über dem Boden angestaut. Ebenso wird mit Reispflanzen verfahren.

Im Vorauflaufverfahren, d. h. 3 - 4 Tage nach dem Anpflanzen werden die Verbindungen ins Bewässerungswasser gegossen (in Form von wäßrigen Suspensionen bzw. Emulsionen) oder in Form von Granulaten ins Wasser gestreut. ...

Jeweils 3 Wochen später wird die herbizide Wirkung und eine eventuelle Schadwirkung gegenüber Reis bonitiert.

Die Ergebnisse zeigten, daß sich die erfindungsgemäßen Verbindungen zur selektiven Unkrautbekämpfung in Reis eignen. Gegenüber bisherigen Reisherbiziden zeichnen sich die erfindungsgemäßen Verbindungen dadurch aus, daß sie zahlreiche, insbesondere auch schwer bekämpfbare Unkräuter, die aus Dauerorganen wie Knollen (Cyperus!) oder Rhizomen keimen, in sehr wirksamen und kleinen Aufwandmengen bekämpfen und den Reis in keiner Weise schädigen.

Tabelle 4

Reistoleranz und herbizide Wirkung der erfindungsgemäßen Verbindungen

| Beispiel-Nr. | Dosis kg a.i./ ha | OS-V | EOA | CYS | CYD | SCH | ECG |
|---|---|---|---|---|---|---|---|
| 2 | 4 | 0 | 5 | 5 | 5 | 5 | 5 |
| | 2 | 0 | 5 | 5 | 5 | 5 | 5 |
| 36 | 4 | 0 | 5 | 5 | 5 | 5 | - |
| | 2 | 0 | 5 | 5 | 5 | 5 | - |
| 38 | 4 | 1 | 5 | 5 | 5 | 5 | - |
| | 2 | 0 | 5 | 5 | 5 | 5 | - |
| 39 | 2 | 0 | 5 | 5 | 5 | 5 | 5 |
| 40 | 4 | 0 | 5 | 5 | 5 | 5 | 5 |
| | 2 | 0 | 5 | 4 | 5 | 5 | 5 |
| 51 | 4 | 0 | 5 | 5 | 5 | 5 | 5 |
| | 2 | 0 | 4 | 5 | 5 | - | 5 |

Abkürzungen

OS-V = Verpflanzter Reis
EOA = Eleocharis acicularis
CYS = Cyperus serotinus
CYD = Cyperus difformis
SCH = Scirpus hotarui
ECG = Echinochloa crus galli
a.i.= Aktivsubstanz

## 4. Wuchshemmung in Getreide

In Schalenversuchen im Gewächshaus wurden junge Getreidepflanzen (Weizen, Gerste und Roggen) im 3-Blatt-Stadium mit den in Tabelle 5 genannten Verbindungen in den angegebenen Wirkstoffkonzentrationen (kg/ha) tropfnaß gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Ergebnisse sind in der Tabelle 5 zusammengefaßt. Bei der Angabe der Wuchshemmung bedeuten 100 % den Stillstand des Wachstums und 0 % ein Wachstum entsprechend dem der unbehandelten Kontrollpflanzen.

...

Tabelle 5

| Beispiel Nr. | Anwendungskonz. (kg/ha) | Wuchshemmung in % | | | phytotox. Wirkung |
|---|---|---|---|---|---|
| | | Weizen | Gerste | Roggen | |
| 28 | 2,5 | 31 | 17 | 31 | keine |
| | 1,25 | 29 | 18 | 25 | Schäden |
| 29 | 2,5 | 23 | 21 | 39 | keine |
| | 1,25 | 24 | 17 | 31 | Schäden |
| 42 | 2,5 | 24 | 20 | 29 | keine |
| | 1,25 | 22 | 19 | 27 | Schäden |
| 46 | 2,5 | 19 | 26 | 27 | keine |
| | 1,25 | 22 | 24 | 28 | Schäden |

5. Wuchshemmung in Wasserreis

Reispflanzen wurden in Kleinparzellen (2 m x 2 m) angezogen und im Stadium der maximalen Bestockung mit den angegebenen Verbindungen behandelt. Die Substanzen können sowohl durch Spritzung appliziert als auch in das Wasser gegeben werden.

3 Wochen nach Behandlung wurde bei allen Pflanzen der Zuwachs gemessen und die Wuchshemmung in % des Zuwachses der Kontrollpflanzen berechnet. Es wurde außerdem auf eine mögliche phytotoxische Wirkung der Verbindungen geachtet.

Die Ergebnisse sind in Tabelle 6 zusammengefaßt. Bei der Angabe der Wuchshemmung bedeuten 100 % den Stillstand des Wachstums und 0 % ein Wachstum entsprechend der Kontrolle.

Tabelle 6

| Beispiel Nr. | Anwendungskonzentration (kg/ha) | Wuchshemmung in % | phytotox. Wirkung |
|---|---|---|---|
| 29 | 1,25<br>0,62 | 21<br>17 | keine Schäden |
| 46 | 1,25<br>0,62 | 24<br>21 | keine Schäden |

6. Fungizide Wirkung gegen Weizenmehltau (Erysiphe graminis)

Weizenpflanzen werden im 3-Blattstadium mit Konidien des Weizenmehltaus (Erysiphe graminis) stark inokuliert und in einem Gewächshaus bei 20 °C und einer relativen Luftfeuchte von 90 - 95 % aufgestellt. 3 Tage nach Inokulation werden die Pflanzen mit den in Tabelle 7 aufgeführten Verbindungen in den Wirkstoffkonzentrationen von 500, 250 und 125 mg/Liter Spritzbrühe tropfnaß gespritzt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle 7 zusammengefaßt.

...

Tabelle 7

| Verbindung nach Beispiel Nr. | mit Weizenmehltau befallene Blattfläche in % bei mg Wirkstoff/l Spritzbrühe 500 | 250 | 125 |
|---|---|---|---|
| 1 | 0 | 0 | 0 |
| 2 | 0 | 0 | 0 |
| 3 | 0 | 0 | 0 |
| 6 | 0 | 0 | 0 - 3 |
| 7 | 0 | 0 | 0 |
| 10 | 0 | 0 | 0 |
| 13 | 0 | 0 | 0 |
| 14 | 0 | 0 | 0 - 3 |
| 15 | 0 | 0 | 0 |
| 16 | 0 | 0 | 0 |
| 17 | 0 | 0 | 0 |
| 18 | 0 | 0 | 0 |
| 20 | 0 | 0 | 0 |
| 21 | 0 | 0 | 0 |
| 22 | 0 | 0 | 0 |
| 24 | 0 | 0 | 0 |
| 25 | 0 | 0 | 0 |
| 26 | 0 | 0 | 0 |
| 27 | 0 | 0 | 0 |
| 28 | 0 | 0 | 0 |
| 29 | 0 | 0 | 0 |
| 31 | 0 | 0 | 0 |
| 32 | 0 | 0 | 0 |
| 35 | 0 | 0 | 0 |
| 36 | 0 | 0 | 0 |
| 37 | 0 | 0 | 0 |
| 38 | 0 | 0 | 0 |
| 39 | 0 | 0 | 0 |
| 40 | 0 | 0 | 0 |
| 42 | 0 | 0 | 0 |

...

Fortsetzung Tabelle 7

| Verbindung nach Beispiel Nr. | mit Weizenmehltau befallene Blattfläche in % bei mg Wirkstoff/l Spritzbrühe 500 | 250 | 125 |
|---|---|---|---|
| 45 | 0 | 0 | 0 |
| 47 | 0 | 0 | 0 |
| 49 | 0 | 0 | 0 |
| 51 | 0 | 0 | 0 |
| 52 | 0 | 0 | 0 |
| 53 | 0 | 0 | 0 |
| 55 | 0 | 0 | 0 |
| 56 | 0 | 0 | 0 |
| 59 | 0 | 0 | 0 |
| 67 | 0 | 0 | 0 |
| 72 | 0 | 0 | 0 |
| 90 | 0 | 0 | 0 |
| 101 | 0 | 0 | 0 |
| unbehandelte infizierte Pflanzen | | 100 | |

## 7. Fungizide Wirkung gegen Gerstenmehltau (Erysiphe graminis sp. hordei)

Gerstenpflanzen werden im 3-Blattstadium mit Konidien des Gerstenmehltaus (Erysiphe graminis sp. hordei) stark inokuliert und in einem Gewächshaus bei 20 °C und einer relativen Luftfeuchte von 90 - 95 % aufgestellt. 3 Tage nach Inokulation werden die Pflanzen mit den in Tabelle 8 aufgeführten Verbindungen in den Wirkstoffkonzentrationen von 500 und 250 mg/Liter Spritzbrühe tropfnaß gespritzt. Nach einer Inkubationszeit von 10 Tagen werden die Pflanzen auf Befall mit Gerstenmehltau untersucht. Der Befallsgrad wird aus-

...

gedrückt in % befallener Blattfläche, bezogen auf ungehandelte, infizierte Kontrollpflanzen (= 100 % Befall). Das Ergebnis ist in der Tabelle 8 zusammengefaßt.

Tabelle 8

| Verbindungen gem. Beispiel Nr. | mit Gerstenmehltau befallene Blattfläche in % bei mg Wirkstoff/Liter Spritzbrühe 500 | 250 |
|---|---|---|
| 2 | 0 | 0 |
| 7 | 0 | 0 |
| 10 | 0 | 0 |
| 16 | 0 | 0 |
| 17 | 0 | 0 |
| 19 | 0 | 0 |
| 21 | 0 | 0 |
| 22 | 0 | 0 |
| 24 | 0 | 0 |
| 25 | 0 | 0 |
| 27 | 0 | 0 |
| 29 | 0 | 0 |
| 31 | 0 | 0 |
| 32 | 0 | 0 |
| 37 | 0 | 0 |
| 39 | 0 | 0 |
| 42 | 0 | 0 - 3 |
| 47 | 0 | 0 |
| 49 | 0 | 0 |
| 51 | 0 | 0 |
| 55 | 0 | 0 - 3 |
| 56 | 0 | 0 |
| 53 | 0 | 0 |
| unbehandelte infizierte Pflanzen | 100 | |

...

8. Fungizide Wirkung gegen Gurkenmehltau (Erysiphe cichoracearum)

Gurkenpflanzen (Sorte Delikateß) werden im 2-Blattstadium mit einer Konidiensuspension von Gurkenmehltau (Erysiphe cichoracearum) stark inokuliert. Nach einer Antrocknungszeit der Sporensuspension von 30 Minuten werden die Pflanzen in einem Gewächshaus bei 22 °C und 90 % relativer Luftfeuchte aufgestellt. 3 Tage nach Infektion werden die Pflanzen mit den in Tabelle 9 genannten Verbindungen und Wirkstoffkonzentrationen tropfnaß gespritzt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall). Das Ergebnis ist in der Tabelle 9 zusammengefaßt.

Tabelle 9

| Verbindungen gemäß Beispiel Nr. | mit Gurkenmehltau befallene Blattfläche in % bei mg Wirkstoff/Liter Spritzbrühe 500 | 250 | 125 |
|---|---|---|---|
| 42 | 0 | 0 | 0 |
| 59 | 0 | 0 | 0 |
| 7 | 0 | 0 | 0 |
| 10 | 0 | 0 | 0 |
| 14 | 0 | 0 | 0 |
| 17 | 0 | 0 | 0 |
| 28 | 0 | 0 | 0 |
| 29 | 0 | 0 | 0 |
| 31 | 0 | 0 | 0 |
| 32 | 0 | 0 | 0 |
| 35 | 0 | 0 | 0 |
| 43 | 0 | 0 | 0 |
| 55 | 0 | 0 | 0 |
| 12 | 0 | 0 | 0 |

...

Fortsetzung Tabelle 9

| Verbindungen gemäß Beispiel Nr. | mit Gurkenmehltau befallene Blattfläche in % bei mg Wirkstoff/Liter Spritzbrühe 500 | 250 | 125 |
|---|---|---|---|
| 13 | 0 | 0 | 0 |
| 15 | 0 | 0 | 0 |
| 19 | 0 | 0 | 0 |
| 68 | 0 | 0 | 0 |
| 45 | 0 | 0 | 0 |
| 47 | 0 | 0 | 0 |
| 49 | 0 | 0 | 0 |
| 56 | 0 | 0 | 0 |
| 11 | 0 | 0 | 0 |
| 16 | 0 | 0 | 0 |
| 53 | 0 | 0 | 0 |
| 67 | 0 | 0 | 0 |
| 34 | 0 | 0 | 0 |
| 3 | 0 | 0 | 0 |
| 5 | 0 | 0 | 0 |
| 4 | 0 | 0 | 0 |
| 66 | 0 | 0 | 0 |
| 1 | 0 | 0 | 0 |
| 21 | 0 | 0 | 0 |
| 2 | 0 | 0 | 0 |
| 22 | 0 | 0 | 0 |
| 24 | 0 | 0 | 0 |
| 25 | 0 | 0 | 0 |
| 26 | 0 | 0 | 0 |
| 27 | 0 | 0 | 0 |
| 92 | 0 | 0 | 0 |
| 36 | 0 | 0 | 0 |
| 37 | 0 | 0 | 0 |
| 38 | 0 | 0 | 0 |
| 39 | 0 | 0 | 0 |
| 40 | 0 | 0 | 0 |

...

Fortsetzung Tabelle 9

| Verbindungen gemäß Beispiel Nr. | mit Gurkenmehltau befallene Blattfläche in % bei mg Wirkstoff/Liter Spritzbrühe 500 | 250 | 125 |
|---|---|---|---|
| 101 | 0 | 0 | 0 |
| 102 | 0 | 0 | 0 |
| 51 | 0 | 0 | 0 |
| 52 | 0 | 0 | 0 |
| 116 | 0 | 0 | 0 |
| unbehandelte infizierte Pflanzen | | 100 | |

9. Fungizide Wirkung gegen Apfelschorf (Venturia inaequalis)

Apfelunterlagen (EM IX) wurden im 4-Blattstadium mit den beanspruchten Verbindungen in den Anwendungskonzentrationen von 500 und 250 mg Wirkstoff/Liter Spritzbrühe tropfnaß behandelt. Nach Antrocknen des Wirkstoffbelages wurden die Pflanzen mit Konidien des Apfelschorfs (Venturia inaequalis) stark infiziert und tropfnaß in eine Klimakammer gestellt, deren Temperatur 22 °C und deren relative Luftfeuchtigkeit 100 % betrug. Nach einer Infektionszeit von 48 Stunden kamen die Pflanzen in ein Gewächshaus mit 18 °C und einer relativen Luftfeuchte von 95 - 100 %. Nach einer Inkubationszeit von 14 Tagen wurden die Pflanzen auf Befall mit Apfelschorf untersucht. Die Beurteilung des Befalls erfolgte wie üblich nach Augenschein. Der Befallsgrad der Pflanzen mit Apfelschorf wurde in % befallener Blattfläche, bezogen auf unbehandelte, infizierte Pflanzen, ausgedrückt und ist in Tabelle 10 wiedergegeben.

...

Tabelle 10

| Verbindungen gemäß Beispiel Nr. | % Schorfbefall bei mg Wirkstoff/ Liter Spritzbrühe 500 | 250 |
|---|---|---|
| 7 | 0 | 0 |
| 18 | 0 | 0 |
| 19 | 0 | 0 |
| 68 | 0 | 0 - 3 |
| unbehandelte, infizierte Pflanzen | 100 | |

10. Fungizide Wirkung gegen den Erreger der Blattfleckenkrankheit der Zuckerrübe (Cercospora beticola)

Zuckerrübenpflanzen wurden im 6-Blattstadium mit den beanspruchten Verbindungen in den Aufwandmengen von 500 und 250 mg/l Spritzbrühe behandelt. Nach Antrocknen des Wirkstoffbelages wurden die Pflanzen mit Konidien des Erregers der Blattfleckenkrankheit der Rübe (Cercospora beticola) stark inokuliert und tropfnaß in eine Klimakammer mit ca. 100 % relativer Luftfeuchte und 25 °C gestellt. 48 Stunden später kamen die Pflanzen in ein Gewächshaus zurück. 14 Tage später wurden sie auf Befall mit der Blattfleckenkrankheit untersucht. Der Befallsgrad wurde in % befallener Blattfläche, bezogen auf unbehandelte, infizierte Kontrollpflanzen (= 100 %), ausgedrückt. Die Ergebnisse sind in Tabelle 11 zusammengestellt.

...

Tabelle 11

| Verbindungen gemäß Beispiel Nr. | mit Cercospora beticola befallene Blattfläche bei mg Wirkstoff/l Spritzbrühe 500 | 250 |
|---|---|---|
| 18 | 0 | 0 |
| 59 | 0 | 0 |
| 7 | 0 | 0 |
| 17 | 0 | 0 - 3 |
| 28 | 0 | 0 - 3 |
| 29 | 0 | 0 |
| 12 | 0 | 0 |
| 72 | 0 | 0 |
| 53 | 0 | 0 |
| 22 | 0 | 0 |
| 24 | 0 | 0 |
| 25 | 0 | 0 |
| 39 | 0 | 0 |
| unbehandelte, infizierte Pflanzen | 100 | |

...

PATENTANSPRÜCHE: 83/F 140

1. Neue 1,2-Diaryl-3-azolyl-propan-Derivate der Formel (I)

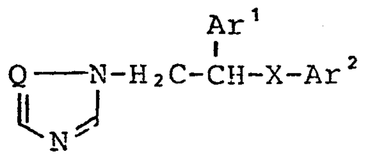 (I)

wobei in dieser Formel

$Ar^1$ und $Ar^2$ gleich oder verschieden sind und die Bedeutung von 1- oder 2-Naphthyl oder einer Gruppe

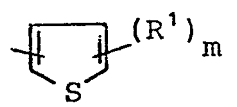 , 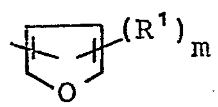 , 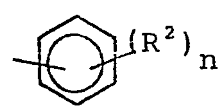

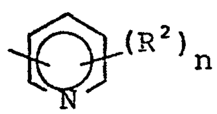

haben, mit

$R^1$ = Wasserstoff, $C_1$-$C_6$-Alkyl oder Halogen,

$R^2$ = Wasserstoff, Halogen, Halogen($C_1$-$C_4$)-alkyl, bevorzugt Trifluormethyl und Trichlormethyl, Nitril, Nitro, Hydroxy, ($C_1$-$C_6$)-Alkyl, ($C_5$-$C_6$)-Cycloalkyl, ($C_1$-$C_6$)-Alkoxy, Halogen($C_1$-$C_4$)-alkoxy, Phenoxy, Phenyl- oder Phenyl($C_1$-$C_4$)-alkyl, wobei die drei letztgenannten Reste bis zu 3 Halogenatome tragen können,

m = 1, 2 oder 3 und
n = 1, 2, 3 oder 4, wobei im Falle m > 1 oder n > 1 die Substituenten verschieden sein können,

X einen Rest der Formeln

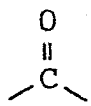 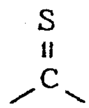 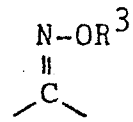 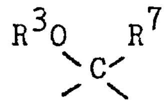 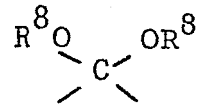

oder

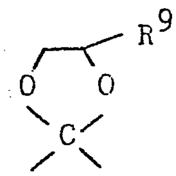

wobei in diesen Gruppierungen

$R^3$ Wasserstoff, $(C_1-C_6)$-Alkyl, $(C_5-C_6)$-Cycloalkyl, $(C_2-C_6)$-Alkenyl, $(C_3-C_6)$-Alkinyl, Phenyl-$(C_1-C_4)$-alkyl, Phenoxy-$(C_1-C_6)$-alkyl, wobei die beiden letztgenannten Gruppen ihrerseits bis zu dreifach halogensubstituiert sein können, oder einen Rest der Formeln

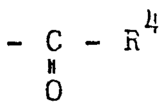 oder 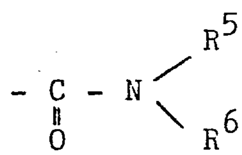 ,

wobei

$R^4$ $(C_1-C_6)$-Alkyl, $(C_5-C_6)$-Cycloalkyl, $(C_2-C_6)$-Alkenyl, Phenyl, Naphthyl oder Phenyl$(C_1-C_4)$-alkyl bedeutet, wobei die drei letztgenannten Reste bis zu dreifach durch Halogen, $(C_1-C_4)$-Alkyl oder $(C_1-C_4)$-Alkoxy oder durch eine

Trifluor- oder Trichlormethylgruppe substituiert sein können,

$R^5$, $R^6$ unabhängig voneinander Wasserstoff, ($C_1$-$C_6$)-Alkyl oder Phenyl, das durch Halogen substituiert sein kann, bedeuten, wobei im Fall $R^5$ oder $R^6$ Wasserstoff oder Phenyl bedeutet, $R^5$ und $R^6$ verschieden sein müssen,

$R^7$ Wasserstoff, ($C_1$-$C_6$)-Alkyl oder Phenyl, das ein bis dreifach halogeniert sein kann, bevorzugt Wasserstoff, wenn $R^3$ nicht Wasserstoff bedeutet,

$R^8$ bei gleicher Bedeutung ($C_1$-$C_6$)-Alkyl und

$R^9$ Wasserstoff, ($C_1$-$C_6$)-Alkyl, halogeniertes ($C_1$-$C_6$)-Alkyl, Phenyl, Naphthyl, Phenyl-($C_1$-$C_4$)-alkyl oder Phenoxy-($C_1$-$C_6$)-alkyl, wobei diese drei letztgenannten Reste ein- bis dreifach halogeniert sein können, und

Q N oder CH, bedeutet, wobei jedoch CH ausgenommen ist, wenn $Ar^1$ Phenyl und zusätzlich X einen Rest der Formel C=O oder CH-$OR^{10}$ bedeutet, worin $R^{10}$ Wasserstoff oder einen Rest der Formel $\underset{\underset{O}{\|}}{C}-R^{11}$ bedeutet, worin $R^{11}$ die Bedeutung von Phenyl, ($C_1$-$C_6$)-Alkyl oder von ($C_1$-$C_6$)-Dialkylamino hat,

sowie ihre Salze, Metallsalzkomplexe und Quaternisierungsprodukte.

2. Verfahren zur Herstellung der Verbindungen der Formel I, dadurch gekennzeichnet, daß man

a) Verbindungen der Formel II,

$$Ar^1 - \underset{\underset{CH_2}{|}}{C} - \overset{\overset{O}{\|}}{C} - Ar^2 \qquad (II)$$

mit Imidazol oder 1,2,4-Triazol unter Zusatz einer Base umsetzt, oder

b) Verbindungen der Formel III,

$$Ar^1 - \underset{\underset{CH_2Cl}{|}}{CH} - \overset{\overset{O}{\|}}{C} - Ar^2 \qquad (III)$$

mit Imidazol oder 1,2,4-Triazol in Gegenwart eines Säurebindemittels umsetzt,

und die so erhaltenen Verbindungen der Formel I mit X= C=O gegebenenfalls durch Derivatisierung der Carbonylgruppe in andere Verbindungen der Formel I umwandelt und die erhaltenen Verbindungen der Formel I in ihre Salze, Metallsalzkomplexe und Quaternisierungsprodukte überführt.

3. Fungizide, herbizide und pflanzenwachstumsregulierende Mittel, gekennzeichnet durch einen wirksamen Gehalt an einer Verbindung der Formel I.

4. Verwendung von Verbindungen der Formel I zur Bekämpfung von Schadpilzen, Schadpflanzen bzw. zur Beeinflussung des Pflanzenwachstums.

5. Verwendung von Verbindungen der Formel I gemäß Anspruch 4 zur Bekämpfung von Schadpflanzen in Reiskulturen.

6. Verfahren zur Bekämpfung von Schadpilzen, Schadpflanzen und zur Beeinflussung des Pflanzenwachstums, dadurch gekennzeichnet, daß man auf die zu behandelnden Pflanzen

oder Anbauflächen eine wirksame Menge einer Verbindung von Formel I aufbringt.

7. Verfahren gemäß Anspruch 6 zur Bekämpfung von Schadpflanzen in Reiskulturen.

PATENTANSPRÜCHE für Österreich:

1. Verfahren zur Herstellung von Verbindungen der Formel I,

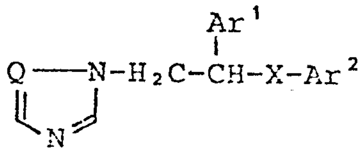 (I)

wobei in dieser Formel

$Ar^1$ und $Ar^2$ gleich oder verschieden sind und die Bedeutung von 1- oder 2-Naphthyl oder einer Gruppe

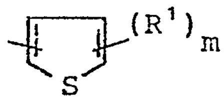 , 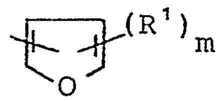 , 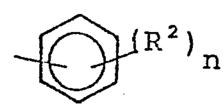

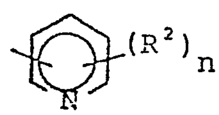

haben, mit

$R^1$ = Wasserstoff, $C_1$-$C_6$-Alkyl oder Halogen,

$R^2$ = Wasserstoff, Halogen, Halogen($C_1$-$C_4$)-alkyl, bevorzugt Trifluormethyl und Trichlormethyl, Nitril, Nitro, Hydroxy, ($C_1$-$C_6$)-Alkyl, ($C_5$-$C_6$)-Cycloalkyl, ($C_1$-$C_6$)-Alkoxy, Halogen($C_1$-$C_4$)-alkoxy, Phenoxy, Phenyl- oder Phenyl($C_1$-$C_4$)-alkyl, wobei die drei letztgenannten Reste bis zu 3 Halogenatome tragen können,

m = 1, 2 oder 3 und
n = 1, 2, 3 oder 4, wobei im Falle m 1 oder n > 1 die Substituenten verschieden sein können,

X einen Rest der Formeln

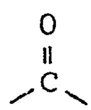 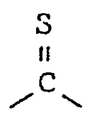 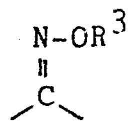 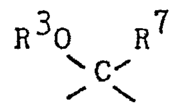 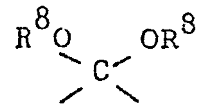

oder

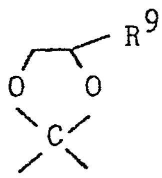

wobei in diesen Gruppierungen

$R^3$ Wasserstoff, $(C_1-C_6)$-Alkyl, $(C_5-C_6)$-Cycloalkyl, $(C_2-C_6)$-Alkenyl, $(C_3-C_6)$-Alkinyl, Phenyl-$(C_1-C_4)$-alkyl, Phenoxy-$(C_1-C_6)$-alkyl, wobei die beiden letztgenannten Gruppen ihrerseits bis zu dreifach halogensubstituiert sein können, oder einen Rest der Formeln

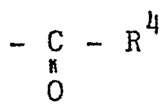 oder 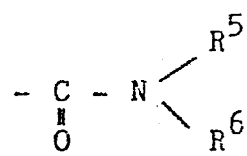 ,

wobei

$R^4$ $(C_1-C_6)$-Alkyl, $(C_5-C_6)$-Cycloalkyl, $(C_2-C_6)$-Alkenyl, Phenyl, Naphthyl oder Phenyl$(C_1-C_4)$-alkyl bedeutet, wobei die drei letztgenannten Reste bis zu dreifach durch Halogen, $(C_1-C_4)$-Alkyl oder $(C_1-C_4)$-Alkoxy oder durch eine

Trifluor- oder Trichlormethylgruppe substituiert sein können,

$R^5$,$R^6$ unabhängig voneinander Wasserstoff, ($C_1$-$C_6$)-Alkyl oder Phenyl, das durch Halogen substituiert sein kann, bedeuten, wobei im Fall $R^5$ oder $R^6$ Wasserstoff oder Phenyl bedeutet, $R^5$ und $R^6$ verschieden sein müssen,

$R^7$ Wasserstoff, ($C_1$-$C_6$)-Alkyl oder Phenyl, das ein bis dreifach halogeniert sein kann, bevorzugt Wasserstoff, wenn $R^3$ nicht Wasserstoff bedeutet,

$R^8$ bei gleicher Bedeutung ($C_1$-$C_6$)-Alkyl und

$R^9$ Wasserstoff, ($C_1$-$C_6$)-Alkyl, halogeniertes ($C_1$-$C_6$)-Alkyl, Phenyl, Naphthyl, Phenyl-($C_1$-$C_4$)-alkyl oder Phenoxy-($C_1$-$C_6$)-alkyl, wobei diese drei letztgenannten Reste ein- bis dreifach halogeniert sein können, und

Q N oder CH, bedeutet, wobei jedoch CH ausgenommen ist, wenn $Ar^1$ Phenyl und zusätzlich X einen Rest der Formel C=O oder CH-$OR^{10}$ bedeutet, worin $R^{10}$ Wasserstoff oder einen Rest der Formel $\underset{\underset{O}{\|}}{C}-R^{11}$ bedeutet, worin $R^{11}$ die Bedeutung von Phenyl, ($C_1$-$C_6$)-Alkyl oder von ($C_1$-$C_6$)-Dialkylamino hat,

dadurch gekennzeichnet, daß man

a) Verbindungen der Formel II,

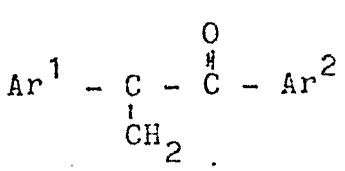

(II)

mit Imidazol oder 1,2,4-Triazol unter Zusatz einer Base umsetzt, oder

b) Verbindungen der Formel III,

$$Ar^1 - \underset{CH_2Cl}{\underset{|}{CH}} - \overset{O}{\overset{\|}{C}} - Ar^2 \qquad (III)$$

mit Imidazol oder 1,2,4-Triazol in Gegenwart eines Säurebindemittels umsetzt,

und die so erhaltenen Verbindungen der Formel I mit X= C=O gegebenenfalls durch Derivatisierung der Carbonylgruppe in andere Verbindungen der Formel I umwandelt und die erhaltenen Verbindungen der Formel I in ihre Salze, Metallsalzkomplexe und Quaternisierungsprodukte überführt.

2. Fungizide, herbizide und pflanzenwachstumsregulierende Mittel, gekennzeichnet durch einen wirksamen Gehalt an einer Verbindung der Formel I.

3. Verwendung von Verbindungen der Formel I zur Bekämpfung von Schadpilzen, Schadpflanzen bzw. zur Beeinflussung des Pflanzenwachstums.

4. Verwendung von Verbindungen der Formel I gemäß Anspruch 3 zur Bekämpfung von Schadpflanzen in Reiskulturen.

5. Verfahren zur Bekämpfung von Schadpilzen, Schadpflanzen und zur Beeinflussung des Pflanzenwachstums, dadurch gekennzeichnet, daß man auf die zu behandelnden Pflanzen oder Anbauflächen eine wirksame Menge einer Verbindung von Formel I aufbringt.

6. Verfahren gemäß Anspruch 5 zur Bekämpfung von Schadpflanzen in Reiskulturen.